# EUROPÄISCHE PATENTANMELDUNG

(11) **EP 1 146 039 A2**
(43) Veröffentlichungstag der Anmeldung: **17.10.2001**
(21) Anmeldenummer: 01108503.2
(22) Anmeldetag: 04.04.2001
(51) Int. Cl.: C07D 207/337, C12P 17/10

(54) **Ein Porphobilinogen-Derivat, sowie Verfahren zur dessen Herstellung**

(30) Priorität: 04.04.2000 DE 10016637
(71) Anmelder: Pichler, Josef, 83737 Irschenberg (DE); Stepp, Herbert, 82152 Planegg (DE); Baumgartner, Reinhold, 85354 Freising (DE); Hofstetter, Alfons, 82008 Unterhaching (DE)
(72) Erfinder: Pichler, Josef, 83737 Irschenberg (DE); Stepp, Herbert, 82152 Planegg (DE); Baumgartner, Reinhold, 85354 Freising (DE); Hofstetter, Alfons, 82008 Unterhaching (DE)
(74) Vertreter: Hofstetter, Alfons J., Dr.rer.nat.

(57) **Zusammenfassung**

Die Erfindung betrifft ein Porphobilinogen-Derivat mit folgender Strukturformel 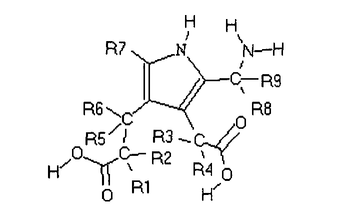 wobei die Reste R1 bis R9 H oder D sind, wobei mindestens einer der Reste R1 bis R9 D ist, wobei das Porphobilinogen-Derivat auch als Salz vorliegen kann. Die Erfindung betrifft außerdem Verfahren zur Herstellung des erfindungsgemäßen Porphobilinogen-Derivats und seine Verwendung zur Herstellung eines Präparats zur Diagnose und/oder Therapie von Tumoren.

## Beschreibung

Die vorliegende Erfindung betrifft ein Porphobilinogen-Derivat, Verfahren zur Herstellung des Porphobilinogen-Derivats und die Verwendung des Porphobilinogen-Derivats zur Herstellung eines Präparats zur Diagnose und/oder Therapie von Tumoren.

5-Aminolävulinsäure hat sich als hervorragendes Kontrastmittel zur Diskriminierung eines Tumors eines Patienten insbesondere in der Urologie, der Neurochirurgie, sowie in der Bronchologie bewährt. Nach Applikation von 5-Aminolävulinsäure reichert sich diese zusammen mit den Folgeprodukten der Porphyrinbiosynthese wie Porphobilinogen und Protoporphyrin-IX besonders in dem Tumorgewebe des Patienten an, das dann mit Hilfe der Fluoreszensspektroskopie nachgewiesen werden kann. Die Verwendung von 5-Aminolävulinsäure eröffnet daher die Möglichkeit einer frühzeitigen und präzisen Diagnose von Krebserkrankungen. Außerdem ist es durch die sogenannte photodynamische Therapie, von beispielsweise malignen Neoplasien, möglich, mit 5-Aminolävulinsäure angereicherte Zellen durch Bestrahlung mit Licht geeigneter Wellenlänge abzutöten und so eine effektive Tumorbehandlung zu erreichen.

Nachteilig ist jedoch, daß die 5-Aminolävulinsäure und ihre biochemischen Folgeprodukte nicht-invasiven diagnostischen Methoden wie beispielsweise der Kemspinresonanz-Tomographie (MRI) nicht zugänglich sind. Ein Nachweis der Anreicherung von 5-Aminolävulinsäure und seinen biochemischen Folgeprodukten ist also nur durch Fluoreszens-Spektroskopie bei direkter Bestrahlung des betreffenden Gewebes möglich. Viele Tumore sind jedoch so positioniert, daß sie nur bei einem operativen Eingriff durch Fluoreszenzspektroskopie nachgewiesen werden können.

Aus der EP 0 845 457 ist weiterhin ein deuteriertes Derivat der 5-Aminolävulinsäure, nämlich Bis-2,2-Deutero-5-Aminolävulinsäure, bekannt, das als Kontrastmittel in der Kemspinresonanz-Tomographie eingesetzt werden kann. Nachteilig ist jedoch, daß 5-Aminolävulinsäure auch in deuterierter Form nicht intravenös applizierbar ist, da 5-Aminolävulinsäure bei neutralem pH polymerisiert. Die intravenöse Applikation ist jedoch besonders vorteilhaft, da durch sie exakt und auch in hohen Dosen dosiert werden kann. Außerdem kann die auf diesem Wege zugeführte Substanz alle Bereiche des Körpers eines Patienten erreichen. Dies ist dann besonders wichtig, wenn beispielsweise ein Tumor in einem Körperbereich lokalisiert ist, der andernfalls nur operativ erreichbar wäre.

Demgemäß ist es Aufgabe der Erfindung eine Verbindung zur Verfügung zu stellen, die und/oder deren Folgeprodukte im Tumorgewebe eines Patienten nach intravenöser Applikation der Verbindung anreicherbar ist/sind und auch durch nicht-invasive diagnostische Methoden, wie beispielsweise durch die Kernspinresonanz-Tomographie detektierbar ist/sind.

Eine erfindungsgemäße Lösung der Aufgabe stellt ein Porphobilinogen-Derivat mit folgender Strukturformel 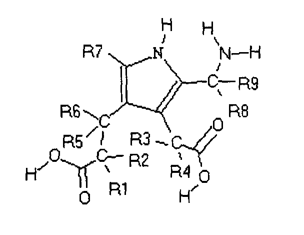 dar, wobei die Reste R1 bis R9 H oder D sind, wobei mindestens einer der Reste R1 bis R9 D ist, wobei das Porphobilinogen-Derivat auch als Salz vorliegen kann. Deuterierte Formen des Porphobilinogens, das biochemisch ein Dimer aus zwei 5-Aminolävulinsäuremolekülen darstellt, werden insbesondere von Tumorzellen zu Protoporphyrin-IX umgewandelt, und es kommt dort nachweislich zu einer Anreicherung des relativ beständigen Protoporphyrin-IX. Das erfindungsgemäße Porphobilinogen-Derivat kann bis zu 9 Deuteriumatome aufweisen, was in der Zelle zu einer enzymatischen Synthese von bis zu 26fach markiertem Protoporphyrin-IX führt, das daher sehr gut insbesondere durch ²H-Kernspinresonanz-Tomographie nachweisbar ist. Der Deuterierungsgrad des Protoporphyrin-IX kann daher gegenüber dem Deuterierungsgrad gesteigert werden, der nach Applikation von deuterierter 5-Aminolävulinsäure zu beobachten ist. So kann durch Applikation von Bis-2.2-Deutero-5-Aminolävulinsäure nur eine 16fach Deuterierung des Protoporphyrin-IX erreicht werden. Eine höhere Deuterierung ist durch Applikation von deuterierter 5-Aminolävulinsäure nicht erreichbar, da nur die Deuterierungen am C-2 der 5-Aminolävulinsäure gegen Wasser stabil sind. Die Deuterierungen des erfindungsgemäßen Porphobilinogen-Derivats sind hingegen gegen Wasser stabil.

In Untersuchungen wurde weiterhin festgestellt, daß die Applikation des Porphobilinogen-Derivats zu einer Anreicherung von Protoporphyrin-IX im Tumorgewebe führt, die die Anreicherung von Protoporphyrin-IX im Tumorgewebe nach Applikation von einem 5-Aminolävulinsäure-Derivat deutlich übersteigt. Das bedeutet, daß durch das erfindungsgemäße Porphobilinogen-Derivat eine sicherere Diagnose bezüglich des Vorhandenseins und der Lokalisation eines Tumors möglich ist als mit einem 5-Aminolävulinsäure-Derivat. Außerdem kann aufgrund der besseren Anreicherung von Protoporphyrin-IX eine wirksamere Tumorbehandlung erfolgen.

Außerdem kann das Porphobilinogen-Derivat als Salz vorliegen, das bei pH 7 stabil ist und daher intravenös applizierbar ist. Dabei finden vor allem die Salze Verwendung, die pharmakologisch unbedenklich einsetzbar sind. Besonders bevorzugt ist ein Porphobilinogen-Derivat, das als Natrium-Salz oder als Kalium-Salz oder als Kalium-, Natrium-Salz vorliegt. Außerdem sind Calciumsalze, Magnesiumsalze, Eisen (II-III)-Salze und Mischsalze (inklusive Hydrogenformen) möglich.

Die obige Aufgabe wird auch gelöst durch ein Verfahren zur Herstellung eines erfindungsgemäßen Porphobilinogen-Derivats, umfassend den folgenden Schritt:
- Synthese des Porphobilinogen-Derivats durch enzymatische Katalyse durch die 5-Aminolävulinsäure-Dehydrase aus
   einem Teil 2-Deutero-5-Aminolävulinsäure oder einem ihrer Salze und/oder Bis-2.2-Deutero-5-Aminolävulinsäure oder einem ihrer Salze und/oder einem weiteren deuterierten 5-Aminolävulinsäure-Derivat oder einem seiner Salze, und aus
   einem zweiten Teil 2-Deutero-5-Aminolävulinsäure oder einem ihrer Salze und/oder Bis-2.2-Deutero-5-Aminolävulinsäure oder einem ihrer Salze und/oder einem weiteren deuterierten 5-Aminolävulinsäure-Derivat oder einem seiner Salze und/oder 5-Aminolävulinsäure oder einem ihrer Salze.

Durch diese Synthese kann ein erfindungsgemäßes Porphobilinogen-Derivat mit bis zu 9 gegen Wasser stabilen Deuterierungen hergestellt werde. Eine solche hohe Deuterierungsstufe kann insbesondere dann erreicht werden, wenn der eine und/oder der zweite für die Synthese verwendete Teil vor der Synthese in D₂O inkubiert wird. Auf diese Weise können durch die Keto-Enol-Tautomerie Deuterierungen in der 5-Aminolävulinsäure erhalten werden, die sich nach der Umsetzung zu Porphobilinogen an gegen Wasser stabilen Positionen befinden. Um zu verhindern, daß bei der Umsetzung noch ein Austausch mit Wasser stattfindet, kann die Synthese des Porphobilinogen-Derivats in D₂O durchgeführt werden.

Die 2-Deutero-5-Aminolävulinsäure oder eines ihrer Salze und/oder die Bis-2.2-Deutero-5-Aminolävulinsäure oder eines ihrer Salze und/oder das weitere deuterierte 5-Aminolävulinsäure-Derivat oder eines seiner Salze kann durch Inkubation von 5-Aminolävulinaldehyd mit D₂O und anschließende Umsetzung zur Säure hergestellt werden.

Alternativ kann das weitere deuterierte 5-Aminolävulinsäure-Derivat oder eines seiner Salze durch Inkubation von 5-Aminolävulinsäure in D₂O hergestellt werden.

Die Herstellung der 2-Deutero-5-Aminolävulinsäure kann auch folgende Schritte umfassen:
- Herstellung von 2-Brom-5-Aminolävulinsäure aus 5-Aminolävulinsäure durch eine Hell-Volhard-Zelinsky-Reaktion;
- Herstellung eines Grignard-Reagenzes aus 2-Brom-5-Aminolävulinsäure durch eine Grignard-Bildungsreaktion; und
- Herstellung von 2-Deutero-5-Aminolävulinsäure aus dem Grignard-Reagenz durch eine Grignard-Deuterierung.

Die auf diese Weise hergestellte 2-Deutero-5-Aminolävulinsäure ist ganz spezifisch am C-2 deuteriert.

Die Herstellung des Bis-2.2-Deutero-5-Aminolävulinsäure kann folgende Schritte umfassen:
- Herstellung von 2-Deutero-2-Brom-5-Aminolävulinsäure aus 2-Deutero-5-Aminolävulinsäure durch eine Hell-Volhard-Zelinsky-Reaktion;
- Herstellung eines weiteren Grignard-Reagenzes aus 2-Deutero-2-Brom-5-Aminolävulinsäure durch eine Grignard-Bildungsreaktion; und
- Herstellung von Bis-2.2-Deutero-5-Aminolävulinsäure aus dem weiteren Grignard-Reagenz durch eine Grignard-Deuterierung.

Alternativ kann die Herstellung des Bis-2.2-Deutero-5-Aminolävulinsäure auch folgende Schritte umfassen:
- Herstellung von 2-Brom-5-Aminolävulinsäure aus 5-Aminolävulinsäure durch eine Hell-Volhard-Zelinsky-Reaktion;
- Herstellung von Bis-2.2-Brom-5-Aminolävulinsäure aus 2-Brom-5-Aminolävulinsäure durch eine Hell-Volhard-Zelinsky-Reaktion;
- Herstellung eines Grignard-Reagenzes aus Bis-2.2-Brom-5-Aminolävulinsäure durch eine Grignard-Bildungsreaktion; und
- Herstellung von Bis-2.2-Deutero-5-Aminolävulinsäure aus dem Grignard-Reagenz durch eine Grignard-Deuterierung.

Die obige Aufgabe wird auch durch ein weiteres Verfahren zur Herstellung eines erfindungsgemäßen Porphobilinogen-Derivats gelöst, das folgende Schritte umfaßt:
- Synthese von mindestens einfach deuteriertem 4-Keto-5-Hydroxyimino-Hexanal-Ester aus 2-Furanethansäureester durch Umkehrung der Paal-Knorr-Synthese in essigsaurer Lösung und durch Zugabe von Schwefelsäure, wobei die Umsetzung in D₂O durchgeführt wird und/oder nach der Umsetzung eine Inkubation in D₂O durchgeführt wird;
- Synthese von mindestens einfach deuterierter 2-(Keto-ethylamin)-Butanalsäure durch eine Stobbe-Kondensation von Bernsteinsäureester mit Aminoethanal mittels Katalyse von Kalium-tert-butylat und anschließender Hydrierung und Ketosierung und Deuterierung durch D₂O; oder Synthese von mindestens einfach deuterierter 2-(Keto-ethylamin)-Butanalsäure durch Ringspaltung von 3-Piperidin-2,5-Dionsäure und Deuterierung durch D₂O; und
- Synthese von mindestens einfach deuteriertem Porphobilinogen-Derivat aus der mindestens einfach deuterierten 2-(Keto-ethylamin)-Butanalsäure und dem mindestens einfach deuterierten 4-Keto-5-Hydroxyimino-Hexanal-Ester durch die Knorrsche Pyrrolsynthese.

Ob die Synthese von mindestens einfach deuterierter 2-(Keto-ethylamin)-Butanalsäure nun durch die erstgenannte Möglichkeit oder durch die zweitgenannte Möglichkeit hergestellt wird, hängt insbesondere von der Verfügbarkeit der Ausgangsprodukte ab.

Erfindungsgemäß kann das Porphobilinogen-Derivat zur Herstellung eines Präparates zur Diagnose und/oder Therapie von Tumoren verwendet werden. Dabei kann das Präparat insbesondere zur Diskriminierung von Tumor- und Normalgewebe durch Fluoreszens-Spektroskopie verwendbar sein, dar das Porphobilinogen-Derivat und seine biochemischen Folgeprodukte im Tumorgewebe angereichert werden. Das Präparat kann jedoch auch zur Diskriminierung von Tumor- und Normalgewebe durch ²H-Kernspinresonanz-Spektroskopie verwendbar sein. Durch ²H-Kernspinresonanz-Spektroskopie (MRI) können auch Tumore identifiziert werden, die aufgrund ihrer Lage nur nach einem operativen Eingriff fluoreszenzspektroskopisch nachgewiesen werden könnten. Das Präparat kann auch zur Diskriminierung von Tumor- und Normalgewebe durch Massenspektroskopie eingesetzt werden, da die deuterierten Substanzen schwerer sind als die undeuterierten Substanzen. Die deuterierten Substanzen sind außerdem durch Röntgendiagnostik detektierbar, so daß das Präparat hier als Kontrastmittel eingesetzt werden kann.

Das Präparat kann insbesondere auch zur photodynamischen Therapie von Tumoren verwendbar sein, wodurch eine unmittelbare Therapie eines diagnostizierten Tumors ermöglicht wird.

Das Präparat kann insbesondere auch zur Therapie von Tumoren durch Gammastrahlen, insbesondere Gammastrahlen von 2.2 MeV, verwendbar sein. Die Gammastrahlung kann dazu durch mehrfache Bestrahlung aus verschiedenen Winkeln auf das Tumorgewebe fokusiert werden: Deuterium wird hierbei mit einem großen Wirkungsgrad zu zellzerstörenden Neutronen und Protonen umgesetzt. Als experimentelle Gammaquelle dient ³⁰Phosphor, und optimal ist der Beschuß von Wasserstoff (Wasser) mit thermischen Neutronen. Die Zellschädigung von normalem Gewebe ist bei den hochenergetischen Strahlungsquellen deutlich geringer als bei den konventionellen Bestrahlungsmethoden. Die zellzerstörende Wirkung der Protonen und Neutronen ist ca. 10-20 mal höher als die der eingesetzten Gammastrahlung und lokal weitgehend begrenzt.

Dadurch, daß bei neutralem pH stabile Salze des erfindungsgemäßen Porphobilinogen-Derivats gewonnen werden können, ist das Präparat ohne weiteres intravenös applizierbar. Daneben kann das Präparat auch topisch und/oder oral applizierbar sein.

Weitere Einzelheiten, Merkmale und Vorteile der Erfindung ergeben sich aus der folgenden Beschreibung von Ausführungsbeispielen.

Es zeigen
- Fig. 1: eine schematische Darstellung eines ersten Herstellungsverfahrens eines erfindungsgemäßen Porphobilinogen-Derivats;
- Fig. 2: schematisch dargestelltes Verfahren zur Herstellung von 2-Deutero-5-Aminolävulinsäure; und
- Fig. 3: eine schematische Darstellung eines zweiten Herstellungsverfahrens eines erfindungsgemäßen Porphobilinogen-Derivats.

In den Figuren wird Aminolävulinsäure kurz ALA genannt.

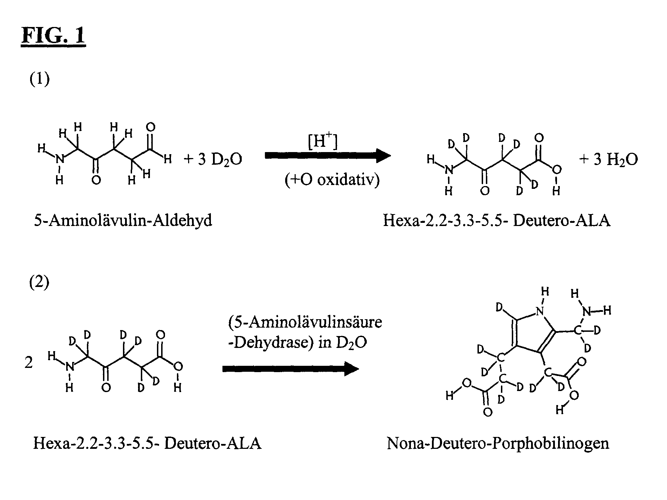

In Fig. 1 ist ein möglicher Syntheseweg für die Herstellung eines erfindungsgemäßen Porphobilinogen-Derivats dargestellt. In einem ersten Schritt wird aus 5-Aminolävulin-Aldehyd (4-Keto-5-Amino-Pentanal) mit überschwerem Wasser im Überschuß über die Keto-Enol-Tautomerie eine deuterierte Form hergestellt, die schließlich zu Hexa-2.2-3.3-5.5-Deutero-5-Aminolävulinsäure umgesetzt wird. Je nach Grad des Austauschs können beispielsweise auch Penta-2-3.3-5.5-Deutero-5-ALA oder Tetra-3.3-5.5-Deutero-5-ALA synthetisiert werden. Hexa-2.2-3.3-5.5-Deutero-5-Aminolävulinsäure läßt sich enzymatisch durch die Katalyse der 5-Aminolävulinsäure-Dehydrase in einem zweiten Schritt zu Nona-Deutero-Porphobilinogen umsetzen.

In Schritt 2 können auch auf andere Art und Weise hergestellte deuterierte 5-Aminolävulinsäure-Derivate eingesetzt werden. Beispielsweise könnten auch 2-Deutero-5-Aminolävulinsäure und/oder Bis-2.2-Deutero-5-Aminolävulinsäure eingesetzt werden, deren Herstellung im folgenden beschrieben wird. Insbesondere durch den Einsatz von Bis-2.2-Deutero-5-Aminolävulinsäure kann nach Inkubation in D₂O durch Schritt 2 ebenfalls Nona-Deutero-Porphobilinogen hergestellt werden. Dieses Porphobilinogen-Derivat ist nun als Salz einem Patienten applizierbar, insbesondere intravenös applizierbar. In vivo werden 4 Moleküle des Porphobilinogen-Derivats in Protoporphyrin IX umgewandelt, das daher hoch deuteriert ist und deshalb leicht durch nicht-invasive Methoden wie durch die ²H-Kemspinresonanz-Spektroskopie nachgewiesen werden kann.

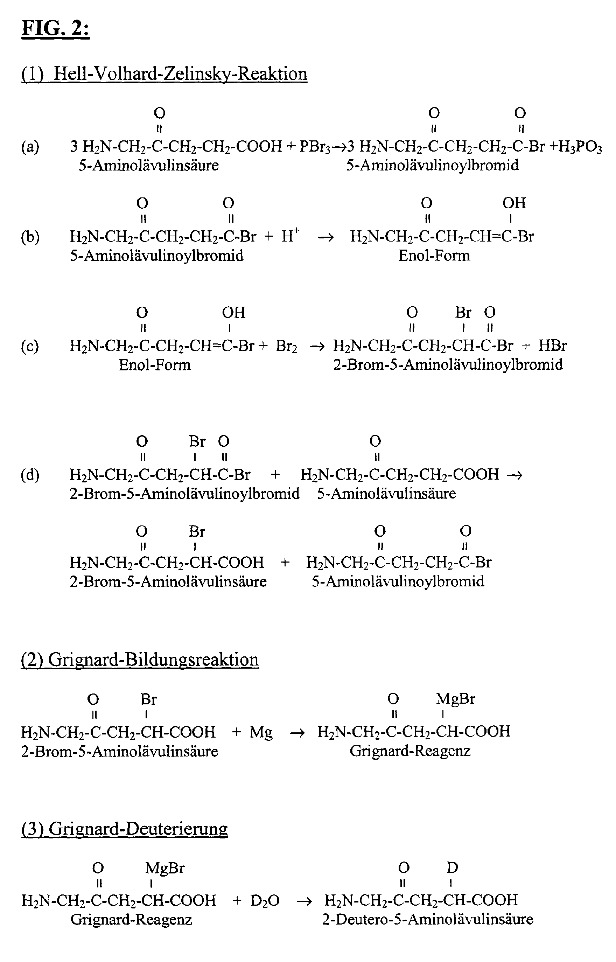

Die Fig. 2 zeigt die Synthese von 2-Deutero-Aminolävulinsäure bzw. einem ihrer Salze mit auftretenden Zwischenprodukten. In einer ersten Reaktion, einer sogenannten Hell-Volhard-Zelinsky-Reaktion wird zunächst 5-Aminolävulinsäure zu 5-Aminolävulinoylbromid umgesetzt. Die Ketogruppe muß hierfür besonders geschützt werden. Als Initiator wird PBr₃ benötigt, das entweder direkt eingesetzt wird oder durch Zugabe von Phosphor und Brom unter den Reaktionsbedingungen gebildet wird. 5-Aminolävulinoylbromid enolisiert bei Säurekatalyse außerordentlich rasch. Die entstandene Enol-Form wird dann in einem weiteren Schritt zu 2-Brom-5-Aminolävulinoylbromid bromiert, das dann eine Austauschreaktion mit der noch unveränderten 5-Aminolävulinsäure eingeht. Dabei entstehen 2-Brom-5-Aminolävulinsäure und 5-Aminolävulinoylbromid. Letzteres tritt erneut in einen Reaktionszyklus ein. 2-Brom-5-Aminolävulinsäure wird anschließend gereinigt. Für die Herstellung von Bis-2.2-Deutero-5-Aminolävulinsäure kann 2-Brom-5-Aminolävulinsäure erneut einer Hell-Volhard-Zelinsky-Reaktion unterworfen werden, wodurch Bis-2.2-Brom-5-Aminolävulinsäure synthetisierbar ist.

Als zweite Reaktion schließt sich eine Grignard-Bildungsreaktion an, bei der 2-Brom-5-Aminolävulinsäure oder Bis-2.2-Brom-5-Aminolävulinsäure (in Fig. 2 nicht dargestellt) in ein Grignard-Reagenz umgewandelt wird. Als Lösungsmittel wird hierbei trockener Ether verwendet wie beispielsweise Diethylether oder Tetrahydrofüran. Da Grignard-Reagenzien heftig mit verschiedenen funktionellen Gruppen (wie Carbonylgruppe, Aminogruppe etc.) reagieren, sind diese durch geeignete Schutzgruppen vor ungewünschten Reaktionen mit dem synthetisierten Grignard-Reagenz zu schützen. Durch eine Grignard-Deuterierung, die die dritte Reaktion der Synthese darstellt, kann durch Reaktion des jeweiligen Grignard-Reagenzes mit D₂O 2-Deutero-5-Aminolävulinsäure oder Bis-2.2-Deutero-5-Aminolävulinsäure (in Fig. 2 nicht gezeigt) hergestellt werden. Aus 2-Deutero-5-Aminolävulinsäure läßt sich durch wiederholte Durchführung der ersten, zweiten und dritten Reaktion Bis-2.2-Deutero-5-Aminolävulinsäure herstellen.

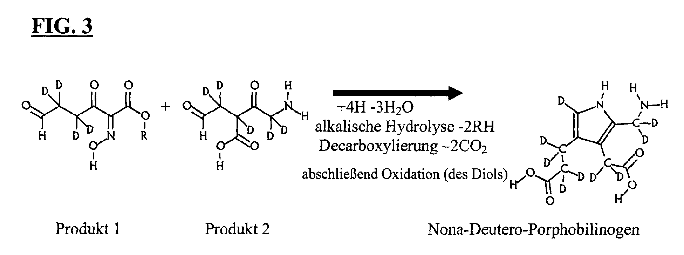

Fig. 3 zeigt eine alternative Herstellung eines erfindungsgemäßen Porphobilinogen-Derivats. Bei der hier dargestellten Reaktion werden 4-Keto-5-Hydroxyimino-Tetra-2.2-3.3-Deutero-Hexanal-Ester (Produkt 1) und 2-(Bi-Deutero-Keto-ethylamin)-2.3.3-Tri-Deutero-Butanalsäure (Produkt 2) eingesetzt und schließlich zu Nona-Deutero-Porphobilinogen umgesetzt.

4-Keto-5-Hydroxyimino-Tetra-2.2-3.3-Deutero-Hexanal-Ester (Produkt 1) ist in Umkehrung zur Paal-Knorr-Synthese in essigsaurer Lösung nach Zugabe von Schwefelsäure verfügbar aus 2-Furanethansäureester (Verfügbar aus Furanal). Die Deuterierung mit schwerem Wasser (D₂O) kann bereits bei diesem Schritt erfolgen. 4-Keto-5-Hydroxyimino-Tetra-2.2-3.3-Deutero-Hexanal-Ester muß sofort nach seiner Bildung weiterverarbeitet werden. Für die Synthese des 4-Keto-5-Hydroxyimino-Tetra-2.2-3.3-Deutero-Hexanal-Esters werden beispielsweis 5 ml 2-Furanethansäureethylester, 0.1 ml Essigsäure und 0.1 ml Schwefelsäure eingesetzt. Die Lösung wird ca. 1 Stunde- 4 Tage bei 20-90 Grad Celsius inkubiert und abschließend in einer Deuterierungsreaktion in D₂O inkubiert.

2-(Bi-Deutero-Keto-ethylamin)-2.3.3-Tri-Deutero-Butanalsäure (Produkt 2) ist verfügbar über die Stobbe-Kondensation von Bernsteinsäureester mit Aminoethanal mittels Katalyse von Kalium-tert-butylat (Johnson 1944) und anschließender Hydrierung und Ketoisierung oder durch Ringspaltung von 3-Piperidin-2,5-Dionsäure. Anschließend folgt eine Deuteriung mit D₂O. Die Herstellung der 2-(Bi-Deutero-Keto-ethylamin)-2.3.3-Tri-Deutero-Butanalsäure (Produkt 2) kann beispielsweise durch folgendes Verfahren erreicht werden:

Nach der Mischung von 5 ml 3-Piperidin-2,5-Dionsäure, 0.1 ml Essigsäure, 0.1 ml Schwefelsäure und 5 ml Ethanol, wird die Lösung ca. 1 Stunde- 4 Tage bei 20-40 Grad Celsius inkubiert, bevor sie wiederum ca. 1 Stunde- 4 Tage auf 70-90 Grad erhitzt wird. Abschließend wird eine Deuterierung mit D₂O durchgeführt, so daß als Produkt 2-(Bi-Deutero-Keto-ethylamin)-2.3.3-Tri-Deutero-Butanalsäure entsteht.

Die in Fig. 3 dargestellte Umsetzung erfolgt nun beispielsweise durch Mischung von 2 ml Produkt 1, 1g KNO₂ und 5 ml HCl, konz. Nach ca. 1 Stunde- 4 Tage bei 20-40 Grad Celsius wird Produkt 2 und 2g Zn-Pulver fraktioniert hinzugeben. Die Lösung wird schließlich ca. 1 Stunde- 1 Tag bei 20-90 Grad Celsius inkubiert. Anschließend werden 2g KOH zugegeben und die Lösung wird zunächst ca. 1 Stunde- 1 Tag bei 20-40 Grad Celsius inkubiert und schließlich ca. 6 Stunden- 1 Tag bei 80-90 Grad Celsius zur Decarboxylierung belassen. Danach wird tropfenweise 2g KMNO₄ (Entfärbungsreaktion) zugegeben. Eine Carboxylierung erfolgt in ca. 2 Stunden- 4 Stunden bei 10-20 Grad Celsius. Nach einer Fällung von Braunstein und Zink wird der Überstand mit HCl angesäuert und die Lösung bis zum Ausfall von Kaliumhydrogenporphobilinogenat konzentriert. Es folgt eine fraktionierte Reinigung des Porphobilinogenat-Derivats und ein massenspektroskopischer Nachweis des Porphobilinogen-Derivats.

## Patentansprüche

1. Porphobilinogen-Derivat mit folgender Strukturformel 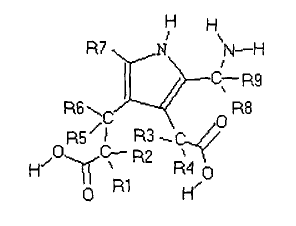 wobei die Reste R1 bis R9 H oder D sind, wobei mindestens einer der Reste R1 bis R9 D ist, wobei das Porphobilinogen-Derivat auch als Salz vorliegen kann.

2. Porphobilinogen-Derivat nach Anspruch 1,
**dadurch gekennzeichnet,**
**daß** das Porphobilinogen-Derivat als Natrium-Salz oder als Kalium-Salz oder als Kalium-, Natrium-Salz vorliegt.

3. Verfahren zur Herstellung eines Porphobilinogen-Derivats gemäß Anspruch 1, umfassend den folgenden Schritt:
- Synthese des Porphobilinogen-Derivats durch enzymatische Katalyse durch die 5-Aminolävulinsäure-Dehydrase aus
einem Teil 2-Deutero-5-Aminolävulinsäure oder einem ihrer Salze und/oder Bis-2.2-Deutero-5-Aminolävulinsäure oder einem ihrer Salze und/oder einem weiteren deuterierten 5-Aminolävulinsäure-Derivat oder einem seiner Salze, und aus
einem zweiten Teil 2-Deutero-5-Aminolävulinsäure oder einem ihrer Salze und/oder Bis-2.2-Deutero-5-Aminolävulinsäure oder einem ihrer Salze und/oder einem weiteren deuterierten 5-Aminolävulinsäure-Derivat oder einem seiner Salze und/oder 5-Aminolävulinsäure oder einem ihrer Salze.

4. Verfahren nach Anspruch 3,
**dadurch gekennzeichnet,**
**daß** der eine und/oder der zweite für die Synthese verwendete Teil vor der Synthese in D₂O inkubiert wird.

5. Verfahren nach einem der Ansprüche 3 oder 4,
**dadurch gekennzeichnet,**
**daß** die Synthese des Porphobilinogen-Derivats in D₂O durchgeführt wird.

6. Verfahren nach einem der Ansprüche 3 bis 5,
**dadurch gekennzeichnet,**
**daß** die 2-Deutero-5-Aminolävulinsäure oder eines ihrer Salze und/oder die Bis-2.2-Deutero-5-Aminolävulinsäure oder eines ihrer Salze und/oder das weitere deuterierte 5-Aminolävulinsäure-Derivat oder eines seiner Salze durch Inkubation von 5-Aminolävulin-Aldehyd mit D₂O und anschließende Umsetzung zur Säure hergestellt wird.

7. Verfahren nach einem der Ansprüche 3 bis 6,
**dadurch gekennzeichnet,**
**daß** das weitere deuterierte 5-Aminolävulinsäure-Derivat oder eines seiner Salze durch Inkubation von 5-Aminolävulinsäure in D₂O hergestellt wird.

8. Verfahren nach Anspruch einem der Ansprüche 3 bis 7,
**dadurch gekennzeichnet,**
**daß** die Herstellung der 2-Deutero-5-Aminolävulinsäure folgende Schritte umfaßt:
- Herstellung von 2-Brom-5-Aminolävulinsäure aus 5-Aminolävulinsäure durch eine Hell-Volhard-Zelinsky-Reaktion;
- Herstellung eines Grignard-Reagenzes aus 2-Brom-5-Aminolävulinsäure durch eine Grignard-Bildungsreaktion; und
- Herstellung von 2-Deutero-5-Aminolävulinsäure aus dem Grignard-Reagenz durch eine Grignard-Deuterierung.

9. Verfahren nach einem der Ansprüche 3 bis 8,
**dadurch gekennzeichnet,**
**daß** die Herstellung des Bis-2.2-Deutero-5-Aminolävulinsäure folgende Schritte umfaßt:
- Herstellung von 2-Deutero-2-Brom-5-Aminolävulinsäure aus 2-Deutero-5-Aminolävulinsäure durch eine Hell-Volhard-Zelinsky-Reaktion;
- Herstellung eines weiteren Grignard-Reagenzes aus 2-Deutero-2-Brom-5-Aminolävulinsäure durch eine Grignard-Bildungsreaktion; und
- Herstellung von Bis-2.2-Deutero-5-Aminolävulinsäure aus dem weiteren Grignard-Reagenz durch eine Grignard-Deuterierung.

10. Verfahren nach einem der Ansprüche 3 bis 8,
**dadurch gekennzeichnet,**
**daß** die Herstellung des Bis-2.2-Deutero-5-Aminolävulinsäure folgende Schritte umfaßt:
- Herstellung von 2-Brom-5-Aminolävulinsäure aus 5-Aminolävulinsäure durch eine Hell-Volhard-Zelinsky-Reaktion;
- Herstellung von Bis-2.2-Brom-5-Aminolävulinsäure aus 2-Brom-5-Aminolävulinsäure durch eine Hell-Volhard-Zelinsky-Reaktion;
- Herstellung eines Grignard-Reagenzes aus Bis-2.2-Brom-5-Aminolävulinsäure durch eine Grignard-Bildungsreaktion; und
- Herstellung von Bis-2.2-Deutero-5-Aminolävulinsäure aus dem Grignard-Reagenz durch eine Grignard-Deuterierung.

11. Verfahren zur Herstellung eines Porphobilinogen-Derivats nach Anspruch 1, umfassend folgende Schritte:
- Synthese von mindestens einfach deuteriertem 4-Keto-5-Hydroxyimino-Hexanal-Ester aus 2-Furanethansäureester durch Umkehrung der Paal-Knorr-Synthese in essigsaurer Lösung und durch Zugabe von Schwefelsäure, wobei die Umsetzung in D₂O durchgeführt wird und/oder nach der Umsetzung eine Inkubation in D₂O durchgeführt wird;
- Synthese von mindestens einfach deuterierter 2-(Keto-ethylamin)-Butanalsäure durch eine Stobbe-Kondensation von Bernsteinsäureester mit Aminoethanal mittels Katalyse von Kalium-tert-butylat und abschließender Hydrierung und Ketosierung und Deuterierung durch D₂O;
oder Synthese von mindestens einfach deuterierter 2-(Keto-ethylamin)-Butanalsäure durch Ringspaltung von 3-Piperidin-2,5-Dionsäure und Deuterierung durch D₂O; und
- Synthese von mindestens einfach deuteriertem Porphobilinogen-Derivat aus der mindestens einfach deuterierten 2-(Keto-ethylamin)-Butanalsäure und dem mindestens einfach deuterierten 4-Keto-5-Hydroxyimino-Hexanal-Ester durch die Knorrsche Pyrrolsynthese.

12. Verwendung des Porphobilinogen-Derivats nach Anspruch 1 zur Herstellung eines Präparates zur Diagnose und/oder Therapie von Tumoren.

13. Verwendung nach Anspruch 12, **dadurch gekennzeichnet, daß** das Präparat zur Diskriminierung von Tumor- und Normalgewebe durch Fluoreszens-Spektroskopie und/oder durch ²H-Kernspinresonanz-Spektroskopie und/oder durch Massenspektroskopie und/oder durch Röntgendiagnostik verwendbar ist.

14. Verwendung nach einem der Ansprüche 12 oder 13, **dadurch gekennzeichnet, daß** das Präparat zur photodynamischen Therapie von Tumoren und/oder zur Therapie von Tumoren durch Gammastrahlen, insbesondere Gammastrahlen von 2.2 MeV, verwendbar ist.

15. Verwendung nach einem der Ansprüche 12 bis 14,
**dadurch gekennzeichnet,**
**daß** das Präparat topisch und/oder oral und/oder intravenös applizierbar ist.
